(19) Europäisches Patentamt
European Patent Office
Office européen des brevets

(11) **EP 3 027 163 B1**

(12) **EUROPEAN PATENT SPECIFICATION**

(45) Date of publication and mention
of the grant of the patent:
**30.11.2022 Bulletin 2022/48**

(21) Application number: **14744848.4**

(22) Date of filing: **30.07.2014**

(51) International Patent Classification (IPC):
*A61K 6/891* (2020.01)  *A61K 6/71* (2020.01)
*A61K 6/802* (2020.01)  *A61K 6/898* (2020.01)

(52) Cooperative Patent Classification (CPC):
(C-Sets available)
**A61K 6/71; A61K 6/802; A61K 6/891; A61K 6/898**

(Cont.)

(86) International application number:
**PCT/EP2014/066334**

(87) International publication number:
**WO 2015/014872 (05.02.2015 Gazette 2015/05)**

(54) **COMPOSITION FOR FILLING BONE AND PERIODONTAL DEFECTS**

ZUSAMMENSETZUNG ZUR FÜLLUNG VON KNOCHEN- UND PERIODONTALEN DEFEKTEN

COMPOSITION POUR REMPLIR DES DÉFAUTS OSSEUX ET PARODONTAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2013 IT MI20131300**

(43) Date of publication of application:
**08.06.2016 Bulletin 2016/23**

(73) Proprietor: **Nobil Bio Ricerche S.r.l.**
**25062 Concesio (BS) (IT)**

(72) Inventors:
• **MORRA, Marco**
**I-25062 Concesio (Bergamo) (IT)**
• **CASSINELLI, Clara**
**I-25062 Concesio (Bergamo) (IT)**
• **BOLLATI, Daniele**
**I-25062 Concesio (Bergamo) (IT)**
• **IVIGLIA, Giorgio**
**I-25062 Concesio (Bergamo) (IT)**

(74) Representative: **Long, Giorgio et al**
**Jacobacci & Partners S.p.A.**
**Via Senato, 8**
**20121 Milano (IT)**

(56) References cited:
FR-A1- 2 732 972       US-A- 5 223 029
US-A1- 2012 009 230     US-B1- 6 180 606

• **GEORGIANA MADALINA ET AL: "Spectral Characteristics and Antioxidant Properties of Tannic Acid Immobilized on Collagen Drug-Delivery Systems", REV. CHIM, 1 January 2009 (2009-01-01), XP055109483,**
• **DATABASE EPODOC [Online] EUROPEAN PATENT OFFICE, THE HAGUE, NL; 27 December 1991 (1991-12-27), MITSUI NORIN KK: "NEW GUM BASE COMPOSED OF TEA POLYPHENOL AND COLLAGEN", XP002722165, Database accession no. jph03297352 & DATABASE WPI Week 199207 Thomson Scientific, London, GB; AN 1992-053581 XP002237508, & JP H03 297352 A (MITSUI NORIN KK) 27 December 1991 (1991-12-27)**
• **DATABASE WPI Week 199503 Thomson Scientific, London, GB; AN 1995-018317 XP002722166, & JP H06 304242 A (NITTA GELATIN KK) 1 November 1994 (1994-11-01)**

**(Cont. next page)**

- **ALONSO E ET AL: "SUITABILITY OF WATER/ETHANOL MIXTURES FOR THE EXTRACTION OF CATECHINS AND PROANTHOCYANIDINS FROM VITIS VINIFERA SEEDS CONTAINEDIN A WINERY BY-PRODUCT", SEED SCIENCE AND TECHNOLOGY : PROCEEDINGS OF THE INTERNATIONAL SEED TESTING ASSOCIATION, WAGENINGEN : VEENMAN, NL, vol. 19, 1 January 1991 (1991-01-01), pages 545-552, XP000576291, ISSN: 0251-0952**
- **Jayamathi Govindaraj ET AL: "Therapeutic effects of proanthocyanidins on the pathogenesis of periodontitis--an overview", Indian journal of experimental biology, 1 February 2011 (2011-02-01), pages 83-93, XP055109537, India Retrieved from the Internet: URL:http://www.ncbi.nlm.nih.gov/pubmed/214 28209**
- **Jayamathi Govindaraj ET AL: "Protective effect of proanthocyanidins on endotoxin induced experimental periodontitis in rats", Indian journal of experimental biology, 1 February 2010 (2010-02-01), pages 133-142, XP055109514, India Retrieved from the Internet: URL:http://www.ncbi.nlm.nih.gov/pubmed/204 55322 cited in the application**

Remarks:
The file contains technical information submitted after the application was filed and not included in this specification

(52) Cooperative Patent Classification (CPC): (Cont.)

C-Sets
**A61K 6/891, C08L 89/06**

**Description**

The present invention relates to a composition for filling tissue defects, such as defects due to loss of soft tissue or resorption of bone tissue around natural teeth (periodontal defects) or implant screws (peri-implant defects), to increase the size of the alveolar ridge, to fill extraction sites and to raise the maxillary sinus.

Background of the invention

Dental practice frequently involves filling cavities with functional materials for the purpose of tissue regeneration. The filling of defects due to loss of soft tissue or resorption of bone tissue around natural teeth (periodontal defects) or implant screws (peri-implant defects), to increase the size of the alveolar ridge, to fill extraction sites and to raise the maxillary sinus fall within this category. The clinical purpose of the filling is to promote tissue regeneration, so that the cavity is filled by regenerated natural tissue. Regeneration materials are commonly applied also in case of absence, low density or reduced size of the bone in areas where, for example, a screw implant would need to be inserted in place of the natural root.

Many materials are available on the market for these applications, and are generally in the form of granules, blocks or gel, the latter often dispensed using a syringe. One typical example of granular material is bovine bone, which purified of any components with antigenic action and reduced to granules of size 0.3-2 mm, is widely used in oral surgery. Other products available are phosphate-ceramic based synthetic fillers, such as hydroxyapatite or tricalcium phosphate or calcium sulphate.

The materials mentioned above work mainly via a mechanical action, providing a functional scaffold for cell adhesion (osteoconduction) and subsequent bone regeneration. Other products on the market add a biological action to it by means of molecules capable of stimulating bone regeneration (osteoinduction). Some bovine bone-based materials on the market, for example, are treated so as not to completely eliminate the organic portion, so that the final product still contains collagen molecules, which play a role in new bone formation. There are also synthetic products which collagen or sequences present in the collagen are added to, to favour the interaction with the cellular component, and thus the regeneration. One commercial product used for filling periodontal defects contains amelogenin, a protein component which governs the formation of dental enamel and bone regeneration. Growth factors and other biological molecules with osteoinductive effects are present in some materials commonly used in the sector.

While the products mentioned are of widespread clinical use and reasonably successful, the development of scientific knowledge in the field has indicated possible ways of improvement. In particular, diseases of bone resorption in dentistry, including the periodontal and peri-implant defects mentioned, fall within the osteo-immune category i.e. the result of the interaction between cells of the immune system and the skeletal system.

For natural reasons, the masticatory apparatus provides for the contiguity of an environment rich in bacteria (oral cavity) and soft and skeletal tissue. The cells of the immune system situated in the border area between the oral cavity and underlying tissue are constantly stimulated by contact with bacteria and continually urged to put in place the defence mechanisms which they are responsible for. This stimulation results in the process commonly known as "inflammation". An integral part of the response to the bacterial stimulus is the production of cytokines and chemokines, i.e. the molecular signals which the body enacts to trigger and sustain the inflammatory response, the instrument with which the bacteria are fought. It is now known that some of the cytokines produced in response to the inflammatory stimulus, such as interleukin 1, have a powerful effect in stimulating the formation of osteoclasts, i.e. the cells which deal with bone resorption. The formation of new bone and resorption of existing bone tissue are normally balanced in the body, ensuring so-called bone homeostasis, but in conditions of prolonged inflammatory response, such as those naturally present in the areas where the teeth emerge in the oral cavity and in the peri-implant areas, the pro-osteoclastogenic action of cytokines leads to the onset of resorption phenomena. These phenomena are favoured not only by general factors such as inadequate oral hygiene, but also and especially individual factors, such as genetic aspects in the case of periodontitis or periodontal disease. In the case of peri-implantitis, which by some estimates has an impact even higher than 20% after 10 years of service, an important causative role is thought to be played by the nature of the peri-implant tissue, which loses some of the characteristics of control of the inflammatory response typical of natural tissue.

Besides the stimulation of osteoclastogenesis and bone tissue resorption, prolonged inflammatory stimulation involves destruction of the soft tissue due to so-called oxidative stress, that is, the damage to tissues as a result of loss of control of the defence mechanisms based on reactive oxygen species (ROS), molecular species generated in inflammatory sites to destroy bacteria and infected tissue.

The European patent EP 2 295 089 A2 describes a formulation made by combining a synthetic ceramic filler or natural bone and a hyaluronic acid gel, so as to obtain a malleable gel easy to adapt to the cavity. This invention combines the mechanical properties of inorganic fillers with the bio-stimulating effect of hyaluronic acid, which also ensures the softness and mouldability of the preparation, but does not have anti-inflammatory and antioxidant effects.

The patent EP 2021044 A2 claims a malleable material for bone implant, containing a solid particulate and a mouldable gel, made with bone particles, collagen and an aqueous solution of polysaccharides. This invention combines the mouldability of the polysaccharide gel with the bio-stimulating action of collagen and the supporting effect of the bone, but does not have a specific antioxidant action for preventing osteoclastogenesis and tissue destruction.

The patent documents US2002/0192263 A1, US2003/0206937 A1 and US7785634 describe bone defect graft materials in the form of gel, having gelatine as a carrier and natural bone particulate as a filler. In this case too, some of the functions previously described are present, but activity against the true promoter of the formation of periodontal and peri-implant defects is lacking, i.e. the tissue damage caused by the oxidative and pro-osteoclastogenic stress of the inflammatory response to the presence of bacteria.

A similar lack is found in the materials on the market, which, despite using bio-stimulant materials and being ability to form mouldable and malleable gels, such as collagen, polysaccharides, in particular hyaluronic acid, pectin, alginic acid, synthetic or natural inorganic fillers, lack an antioxidant activity such as to reduce the formation of cytokines.

It would thus be desirable for the filler materials for bone resorption diseases not only to provide the current mechanical action and possible biological action not aimed at controlling the inflammatory response, but also to provide a specific anti-osteoclastogenic action and preventive effect of damage from oxidative stress by exerting an antioxidant action.

Polyphenols, molecules widely distributed in the plant world, have properties of great interest in this area. Procyanidins and proanthocyanidins, complex molecules consisting of different repeating units and present, inter alia, in grapes, have been extensively studied in relation to their ability to remineralise dental tissue. The efficacy thereof has also been shown in the control of the processes of bone resorption by osteo-immunological action, in particular by means of systemic administration in the diet, as reported in J Govindaraj, Emmadi P, Deepalakshmi, Rajaram V, Prakash G, Puvanakrishnan R., Protective effect of proanthocyanidins on endotoxin induced experimental periodontitis in rats. Indian J Exp Biol. 2010 Feb, 48 (2):133-42.

US 5 223 029 A describes hardening materials for medical and dental use, for example, as a bone cement, a cement for dental use, and a root canal sealing material. Specifically disclosed is a hardening material comprising, inter alia, a liquid solution comprising aterocollagen, tannic acid as hardening adjuster, chitosan, carboxymethylchitin and jellan gum; and a ceramic powder agent.

The possibility of using molecules of this type for treatment and tissue regeneration in periodontal and peri-implant defects is obviously of great practical interest. A local use, unlike the systemic approach used in the Govindarai article mentioned, to concentrate the beneficial action of polyphenolic molecules at the resorption site in question would be particularly interesting. Such local use is hindered however by the highly soluble nature of the molecules in question, which entails their rapid removal from the site of use, and the chemical instability of many of these compounds.

In addition, polyphenols are complex systems and present in low percentages in original plant species. Each fractionation results in a substantial increase in cost and may lead to the practical inapplicability of the interesting general principles set out above.

Summary of the invention

One purpose of the present invention is a composition for use in dentistry having both an anti-inflammatory and anti-osteoclastogenic action assisting bone and tissue regeneration.

A second purpose is a filler material for bone defects containing this composition and having mechanical support and biological stimulation properties.

A further purpose of the invention is a method for making such composition and filler material from an economical source of polyphenolic molecules and an inexpensive and simple extraction process.

These and other purposes are achieved by means of a composition, a filler material and a preparation method thereof as outlined in the appended claims, the definitions of which form an integral part of the present description.

The purposes of this invention are thus achieved by means of a composition, and a relative preparation method, which involves the use of extracts from wine-making production residues, in particular grape seeds and skins. As production residues, the raw material does not present substantial costs. It is known in the art that polyphenols in complex mixtures may be easily and economically extracted from residues of this nature for example with the use of aqueous ethanol solutions (John Shi, Jianmei Yu, Joseph Pohorly, J. Christopher Young, Mike Bryan, Ying Wu, Optimization of the extraction of polyphenols from grape seed meal by aqueous ethanol solution, Food, Agriculture & Environment Vol.1 (2): 42-47. 2003). These extracts contain gallic acid, procyanidins, catechin, epicatechin, epicatechin gallate. As said, these extracts cannot be used as is for local use, since they are water-soluble and unstable. The possibility of local use is achieved according to the present invention by combining the hydroalcoholic extracts, in their original extraction solution or after drying, with natural molecules having a bio-stimulant function or inert carriers. In particular, the extracts can be combined with collagen exploiting the cross-linking effect exerted by some of these molecules, in particular the so-called tannins, or molecules of high molecular weight obtained by the condensation of the repeat unit of flavanol. It has been seen that the cross-linking action takes place directly by means of the hydroalcoholic extracts, without the need for further separations or processes, thus in a simple and inexpensive manner. The gel thus obtained is stable in an aqueous environment, but surprisingly is able to exert the effects of polyphenolic compounds for a prolonged time, circumventing the problems of the high solubility and instability of polyphenolic molecules. This way, the bio-stimulating action of collagen on bone regeneration is combined with the anti-inflammatory and protective effects of polyphenols providing a product with innovative features and specifically suitable for use.

The gel thus obtained is combined with pectin, to improve to a surprising extent the manageability and consistency of the invention, making it particularly adaptable to the cavity which must be filled.

Furthermore, as shown in the application examples presented below, the gel can be combined with granular ceramic fillers, giving rise to a formulation ideally suited to applications providing for the maintenance of space, such as the filling of peri-implant bone defects. This formulation thus provides, in a medium easy to model and apply, the mechanical scaffolding effect of the ceramic granulate, supplemented by the biological and pro-osteogenic action of collagen and anti-inflammatory, antioxidant and anti-osteoclastogenic action of the polyphenols.

Brief description of the drawings

Figure 1 is a graph showing the gene expression for MCB-1, IL6 and IL1β in macrophage cell cultures treated with the polyphenolic extracts;

Figure 2 is a diagram showing the percentage reduction in absorbance at 518 nm in reduction experiments of DPPH;

Figure 3 is a diagram showing the percentage reduction of absorbance at 518 nm in reduction experiments of DPPH with the composition of sample A and compositions of the state of the art;

Figure 4 shows a diagram showing the amounts of polyphenols that are released in PBS after 24 hr + 3 days;

Figure 5 shows a diagram reporting the evaluation of antioxidant activity of the released polyphenols in PBS after 24 hr + 3 days;

Figure 6 shows a diagram reporting measures of genic expression as in example 1, by putting J774A1 cells into contact with aliquots of solutions containing the released material and evaluating the expression of the gene iNOS.

Description of the invention

The present invention relates to a composition for the preparation of a filler material for bone defects, in particular in the dental field, wherein said composition comprises a collagen gel cross-linked with polyphenols in a ceramic support matrix.

The ceramic support matrix may be any matrix, including of natural origin, of the type used as a filler for bone defects, such as powdered bone of animal origin, for example bovine, deprived of the immunogenic components. In certain embodiments, the ceramic support matrix comprises or is composed of ceramic phosphate granules, preferably hydroxyapatite, beta-tricalcium phosphate and mixtures thereof, with dimensions preferably comprised between 50 and 2000 micro metres, more preferably between 100 and 300 micro metres.

In preferred embodiments the polyphenols comprise or consist of polyphenolic mixtures extracted from grape skins and grape seeds, used as is or preferably after the fermentation process and wine-making and drying.

In preferred embodiments said polyphenolic mixtures are hydroalcoholic extracts of the skins and seeds of grapes.

It is, however, possible to use, though less profitable, reconstituted mixtures of polyphenols.

Collagen is preferably type I collagen, of bovine, porcine, equine origin or for recombinant biotechnological production or from plants.

In certain embodiments, the weight ratio between the ceramic matrix (g of granules) and polyphenol extract (g of solution), before drying or lyophilisation, is between 45/55 and 10/90.

The filler material according to the invention contains pectin. This way a malleable filler material is obtained, perfectly suited to the applications listed above.

The composition and the material of the invention can be prepared using a method such as that described below.

Preparation of a polyphenol extract from grape marc

The marc (skins and seeds of grapes from the wine-making process) are first milled, and then extracted using an appropriate solvent whilst in agitation, at ambient temperature and for a time between 30 minutes and two hours. The solvent is preferably a water-alcohol mixture, more preferably a mixture 1:6 water/ethanol.

Preparation of collagen gel cross-linked with polyphenols

The solution of the extract of polyphenols prepared in the previous step is mixed with a solution of collagen as defined above and stirred vigorously until the formation of a gel formed by the cross-linking of collagen with the polyphenols of the extract.

The collagen solution is preferably an aqueous solution and contains between 0.05% and 1% in weight of collagen, more preferably between 0.1% and 0.8% in weight.

Preparation of the filler material according to the invention

The filler material of the invention is obtained by a process which entails:

a) mixing a solution of polyphenol extract obtained as described above with a ceramic support matrix as defined above;
b) drying the mixture of step a) to obtain a granulate;
c) mixing an aqueous solution of collagen as defined above with an aqueous or saline solution of pectin at about pH 5 with an acetic buffer;
d) adding the mixture according to step c) to the granulate of step b);
e) mixing the mixture of step d) to obtain a malleable filler material.

The polysaccharide solution preferably has a concentration in weight of between 3% and 10%, more preferably between 5% and 8%.

The collagen solution preferably has a concentration between 0.05% and 1% in weight, more preferably between 0.1% and 0.8%.

In step c), the weight ratio between the solution of polysaccharides and the collagen solution is preferably between 20:80 and 80:20, more preferably between 40:60 and 60:40.

In step d), the weight ratio between the granules of step b) and the mixture of step c) is preferably between 20:80 and 90:10, more preferably between 50:50 and 80:20.

This composition produces an extremely malleable and workable paste, suitable to be used as a bone filler with improved properties. Indeed, the presence of collagen and polyphenols derived from extracts of Barbera grape promotes the cross-linking effect described above between the two molecules *in situ,* thereby limiting the solubilisation of the antioxidant molecules. The presence of pectin makes it possible to amalgamate the solution, making it malleable and workable, and also acts as a carrier for the gradual release of polyphenols. The ceramic granules provide the mechanical support in the implantation site, aided by the effect of collagen on bone regeneration.

A malleable formulation is thus produced, suitable for locating in the implant site, which combines the classic mechanical support and bio-stimulating action with a specific antioxidant action and reduction of the osteoclastogenesis induced by an inflammatory response.

EXPERIMENTAL PART

Example 1 (for reference only)

Skins and seeds of various grape varieties (Nebbiolo, Barbera), residues of the wine-making process, are ground separately with a mixer to obtain a coarse flour. The flour is placed in contact with a 50% solution of water-ethanol in a 1:6 ratio by weight, and agitated on a shaker for 60 minutes. The extracts are separated from the flour by centrifugation and further filtered with 0.45 and 0.20 micrometre filters.

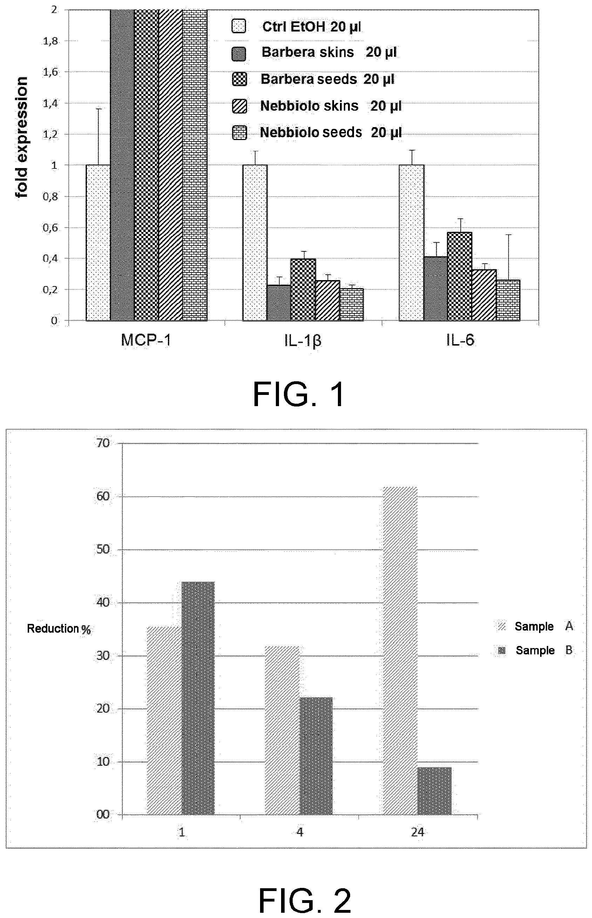

Aliquots of 20 micro litres are placed in macrophage cell cultures J774A1, a control consisting of 20 micro litres of the 50% water-ethanol mixture is also used. After 24h an RT-PCR expression measurement is performed of some genes related to the inflammatory process, the results shown in figure 1 were obtained.

The data shows that all the extracts, without further fractionation and separation, significantly reduce the expression of the two interleukins evaluated. Both are expressed in inflammatory processes and contribute to the generation of osteoclasts which cause bone resorption phenomena. This indicates that the simple hydro-alcoholic extracts from residues of the wine-making process are able to act on the inflammatory cells.

Example 2 (for reference only)

The preparation of Example 1 is repeated with extracts from Nebbiolo grape skins, treated the same way. 5 ml of the hydroalcoholic solution thus obtained is placed in contact with 5 ml of Theracoll porcine collagen, in a 1% solution and vigorously stirred using a Thinky mixer stirrer at 500 revolutions per minute. This operation gives rise to a gel, formed due to the cross-linking of collagen, applicable to periodontal or peri-implant defects using a syringe or spatula.

Example 3 (for reference only)

The gel obtained according to example 2 is mixed with a granulate bone filler consisting of a ceramic matrix made of a mixture of hydroxyapatite and tricalcium phosphate. A filler material is obtained which is easy to use clinically, similarly to existing products, which combines the mechanical support properties of the ceramic filler, the bio-stimulant properties of collagen and anti-inflammatory properties of the extracts from wine-making residues.

The material thus obtained may be dried, so as to form a granulate due to the collapse of the gel, proving entirely similar as regards application and manual dexterity as the granulated products on the market, but combining the mechanical support properties of the ceramic filler, the bio-stimulant properties of the collagen and the anti-inflammatory properties of the polyphenolic extracts.

Example 4

Preparation of malleable filler paste with antioxidant activity: ceramic granules of hydroxyapatite and β-tricalcium phosphate ranging in size between 100 and 300 micro metres are mixed with a hydroalcoholic solution of extracts from Barbera grape skins in 40:60 ratio, obtained as described in Example 1.

The mixture thus obtained is left to dry overnight so as to obtain a granulate in which the extracts are dried on the surface of the particles.

The following are then prepared:

- a pectin solution at a concentration of 7% in weight, in acetate buffer with controlled pH of 5;
- a collagen solution(Theracoll porcine collagen), at a concentration of 0.5% in weight.

The two solutions thus prepared are mixed in a suitable mixer in a ratio of 50:50, the ceramic granulate prepared with hydroalcoholic Barbera extracts dried on the surface is then added to this solution in the ratio of 25:75 and the whole mixed vigorously for 1 min at 2000 rpm.

Example 5

Pastes with different compositions have been prepared.

Initially, ceramic granules of hydroxyapatite and β-tricalcium phosphate having a dimension between 100 and 300 micrometers are mixed with a hydroalcoholic solution of extracts from Barbera grape skins in a 40:60 ratio, obtained as described in example 1. The so obtained mixture is dried overnight so as to give a granulate wherein the extracts are dried on the surface of the particles.

The following samples are prepared:

A) granules prepared as above, to which pectin (Sigma) is added (not according to the invention)

B) granules prepared as above to which porcine collagen (Theracoll) is added (not according to the invention)

C) granules prepared as above to which pectin and collagen are added.

Anyway, the samples are prepared by keeping constant the amount of granules and by adding the other components in a 65:35 ratio.

Evaluation of the antioxidant properties

The evaluation of the antioxidant properties of the invention is performed using the method called DPPH. In this assay the DPPH radical (diphenyl-picrylhydrazyl) is reduced by antioxidant compounds to hydrazine, with consequent alteration of the absorption spectrum which, in the unreduced form, has an absorbance peak at 515-528 nm, which gives the solution a violet colour. The antioxidant action causes the reduction of absorbance at this wavelength and a change in the colour of the solution, which becomes yellow/colourless.

The test is performed by preparing a solution of 300 $\mu$M DPPH in ethanol and adding a known volume of this solution to a known volume of the solutions in ethanol or water of the test compounds.

The test was performed by placing in four different Falcon tubes:

- 1 g of the material prepared in example 4 (sample A; not according to the invention);

- powdered ceramic granules and hydroalcoholic extracts from grape skins prepared as described in the first two paragraphs of example 4, in an amount of 750 mg, equivalent to what is contained in 1 g of material prepared as in example 4 (sample B; not according to the invention);

- 1 g of a material prepared as in example 4, but without the use of hydroalcoholic extracts from grape skins (sample C; not according to the invention);

- powdered ceramic granules in quantities of 750 mg, equivalent to what is contained in 1 g of material prepared as in example 4 (sample D; not according to the invention);

To each of these tubes 5 cc of normal saline solution is added. After 1h, 1 ml of solution is taken from each of the four test tubes and placed in further clean test tubes in order to obtain four samples of 1 cc. 500 $\mu$l of 300 $\mu$M solution of DPPH in ethanol is added to the 4 test tubes.

It is noted that the solution remains purplish in colour in the case of samples that do not contain hydroalcoholic extracts from grape skins (samples C and D). The solutions instead become yellow/ colourless in the case of ceramic granules enriched with hydro-alcoholic extracts from the skins of dried Barbera grapes (sample B) and of the material prepared according to the procedures of example 4 (sample A), which add to the properties described above the specific antioxidant action of the invention, meeting the needs of the application for use, absent in the materials described in the art.

Determination of the antioxidant action of different materials

The two preparations with antioxidant action of the previous example (samples A and B; both not according to the invention) are subjected to the same experiment described above. After 1h, 100 micro litres of supernatant are recovered, which are intended for UV-Visible analysis as described below and the remaining liquid is removed and replaced with fresh liquid. After an additional 3 hours a similar amount of supernatant is recovered and the remaining liquid is removed and replaced with fresh liquid. A similar operation is carried out after a total of 24 hours.

The UV-Visible analysis is performed by adding 500 micro litres of 300 $\mu$M solution of DPPH in ethanol to the 100 micro litres of sample solution and acquiring the absorption spectrum from 480 to 600 nanometres, using a UV-1700 UV-Vis Shimadzu spectrophotometer. The control consists of the samples C and D of the previous experiment, which do not show an antioxidant effect.

The effect of the antioxidant action is expressed as % reduction of the absorbance measured at 518 nm after the addition of 100 micro litres of supernatant from a given sample versus the absorbance measured at 518 nm for the control (granule or gel without antioxidant effect, samples C and D). In practice, the calculation is as follows:

```
% Reduction = 100-A518 sample/A518 reference.
```

The results shown in the diagram in Figure 2 are observed. The data indicate that the simple physical mixture of powders and hydro-alcoholic extracts (Sample B) results in an immediate significant reduction of the DPPH radical, higher than that recorded on the formulate material of sample A (given at 1 h). Already at 4 h, however, the formulation according to the procedures of example 4 is more effective. The release between 4 and 24h still proves remarkably effective for sample A, while the simple physical mixture, for the same times, has by now lost any antioxidant effectiveness. After 24 h, only the sample A is able to exert an antioxidant action on DPPH.

This experiment can be explained by the fact that the simple physical association between ceramic granulate and polyphenolic extracts involves the immediate dissolution of said extracts (wash-out effect), which pass into the solution in high quantities, generating a large antioxidant effect in a short time, but a loss of efficacy already at 4 hours. The association of polyphenolic extracts and other components of the formulation clearly implies a modulation of the rate of release of antioxidant molecules or at least a greater persistence of the antioxidant effect, with obvious advantages for the application, in a surprising and unexpected manner. In fact, while it is known that tannins in the skins of grapes and other plant species can interact with collagen, leading to the formation of gel as described in example 2, it had not been expected that even in the presence of such combination and of the components required for the realisation of the malleable paste described in example 4, a qualitatively superior antioxidant effect would be maintained, for the purposes of the foreseen application, compared to the use of the same amount of extracts without the present formulation.

Comparison of the antioxidant action of compound A and similar products in clinical use.

The experiment of the previous point is repeated to compare the antioxidant action of the material of the invention with that of two similar products in clinical use existing in commerce. In particular, the compound A (not according to the invention) is subjected to the same tests as the previous example, with observation at the experimental times of 1, 4 and 24 hours together with:

Ostim, material for bone regeneration in the form of paste, manufactured by Heraeus Kulzer GmbH, lot 12JA34740, consisting of synthetic hydroxyapatite in aqueous solution,
OsteoBiol Gel 40, material for bone regeneration in the form of paste, lot 130154, produced by Tecnoss srl, made from bovine bone granules and collagen gel.

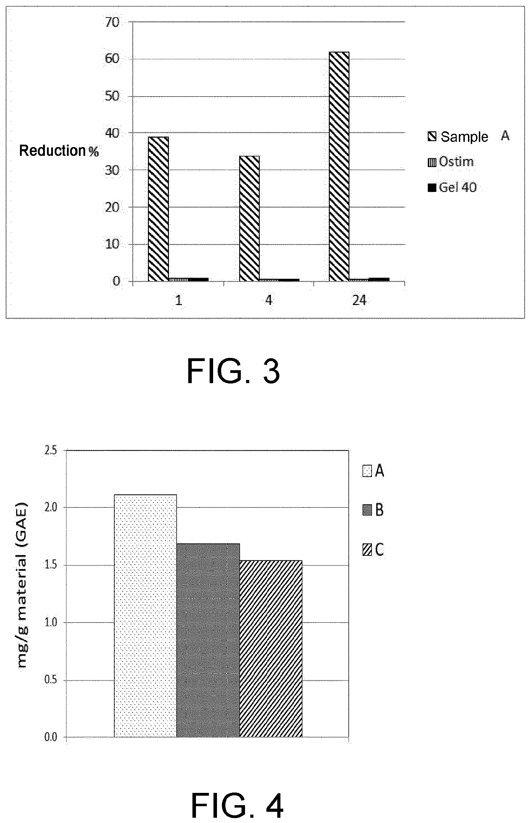

The results obtained, expressed as for the previous example, are shown in Figure 3.
The figure confirms that the formulations available on the market carefully designed to provide the respective characteristics of mechanical support, biological stimulus and mouldability, do not show the antioxidant properties peculiar to the invention; it is also shown that such properties are not given by synthetic materials such as hydroxyapatite or phosphate ceramics or natural materials such as collagen, common to the preparations on the market and to the present invention.

Experiments of comparison of the formulations A), B) and C) of example 5

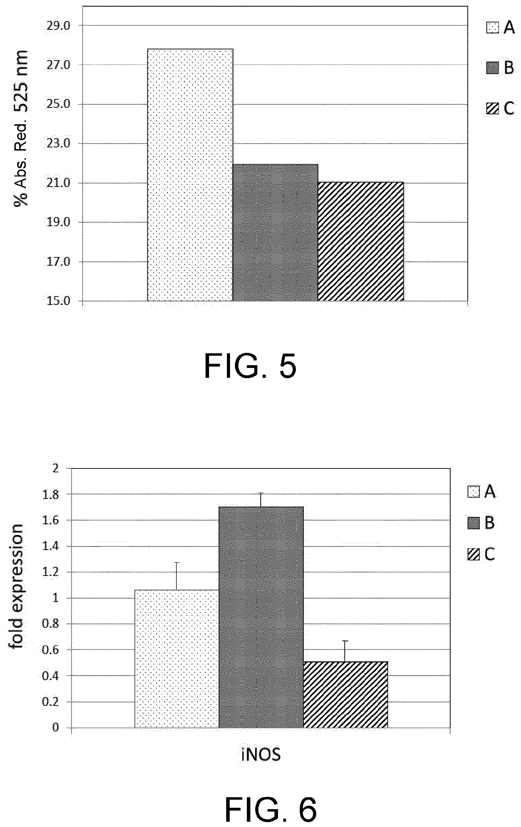

The experiment has been performed by putting the materials A) and B) (not according to the invention) and C) (according to the invention) to release in such an amount to have the same weight of the granules (1 gram). The releasing is performed in PBS, at room temperature, nder stirring at 300 rpm. The release solution is taken after 2 hr and 24 hr and subsequently the materials that had been put to release are incubated in PBS for further 3 days to characterize their long-lasting release. The release solutions were analysed to determine the amount of polyphenols (by means of Folin-Ciocalteu test), the antioxidant activity (by means of DPPH test), the composition (by means of HPLC) and the biological response (by evaluation of the genic expression in inflammatory cells).
The experimental results are shown in figure 4 and show the non-equivalence of the compositions as far as their release is concerned. The amount of polyphenols released by the three formulations is different; only pectin, which is hydrophilic, is not able to retain polyphenols and so it releases them more speedily.
The results of the experiments for the evaluation of the antioxidant activity are shown in figure 5. The antioxidant power after 24 hr + 3 days of release in PBS follows the behaviour of the released polyphenols.
For the evaluation of the antiinflammatory activity, measures of genic expression as in example 1 are performed, by putting J774A1 cells into contact with aliquots of solutions containing the released material. The expression of iNOS gene is evaluated, that is a gene which encodes for an enzyme that produces nitric oxide after induction triggered by inflammatory phenomena. The results shown in figure 6 are obtained.
The data show that the simple formulation of extracts with collagen is less active, in term of suppression of the expression of iNOS, than formulation C). The formulation with pectin is more active than the one with collagen, but as shown in the previous diagrams it releases more polyphenols.
In substance, the formulation with only pectin has not a great capacity to retain polyphenols, as it is known, because it is very hydrophilic. The formulation with only collagen has higher retention capacity, but the released material does not suppress the expression of iNOS, thus showing low antiinflammatory activity. For not understandable reasons, probably due to specific interactions between pectin-collagen and polyphenols, the formulation C) has retention capacity similar of better than collagen alone, but the released material is more active to suppress the inflammatory response than the formulations containing only one of the components. This technical effect, which cannot be explained in the light of the prior art, makes the formulation C) superior with respect to the formulation with collagen. Analogously, it is superior to the formulation with pectin alone, which has an almost immediate release and not suitable mechanical properties because of its hydrophilicity.

**Claims**

1.    A malleable filler material for dental use, said material consisting of:

- a collagen gel cross-linked with polyphenols in a ceramic support matrix, and
- a pectin aqueous or saline solution at about pH 5 with an acetic buffer.

2. The material according to claim 1, wherein the ceramic support matrix consists of ceramic phosphate granules.

3. The material according to claim 2, wherein said ceramic phosphate comprises hydroxyapatite, beta-tricalcium phosphate or mixtures thereof.

4. The material according to any of the claims from 1 to 3, wherein said ceramic support matrix is a granulate with a size between 50 and 2000 micrometres or between 100 and 300 micrometers.

5. The material according to any of the claims from 1 to 4, wherein said polyphenols are selected from polyphenol mixtures extracted from grape skins and grape seeds, used as is or after the fermentation process and wine-making and drying.

6. The material according to claim 5, wherein said polyphenolic mixtures are hydroalcoholic extracts of the skins and seeds of grapes.

7. The material according to any of the claims from 1 to 6, wherein said collagen is collagen type I, of bovine, porcine or equine origin or for recombinant biotechnological production or from plants.

8. The material according to any of the claims from 1 to 7, for use in medicine.

9. The material according to claim 8, for use in filling bone defects, such as defects due to loss of soft tissue or resorption of bone tissue around natural teeth, periodontal defects, or implant screws, peri-implant defects, to increase the size of the alveolar ridge, to fill extraction sites and to raise the maxillary sinus.

10. A method of preparation of the malleable filler material according to any one of claims from 1 to 7, comprising the steps of:

   a) mixing a solution of polyphenol extract obtained from grape marc with a ceramic support matrix;
   b) drying the mixture of step a) to obtain a granulate;
   c) mixing an aqueous solution of collagen with an aqueous or saline solution of pectin at about pH 5 with an acetic buffer;
   d) adding the mixture according to step c) to the granulate of step b);
   e) mixing the mixture of step d) to obtain a malleable filler material.

11. Method according to claim 10, wherein said solution of pectin has a concentration in weight of between 3% and 10%, preferably between 5% and 8%.

12. Method according to any of the claims from 10 to 11, wherein said collagen solution has a concentration between 0.05% and 1% in weight, preferably between 0.1% and 0.8%.

13. Method according to any of the claims from 10 to 12, wherein, in step c), the weight ratio between the solution of pectin and the collagen solution is between 20:80 and 80:20, preferably between 40:60 and 60:40.

14. Method according to any of the claims from 10 to 13, wherein, in step d), the weight ratio between the granulate of step b) and the mixture of step c) is between 20:80 and 90:10, preferably between 50:50 and 80:20.


**Patentansprüche**

1. Verformbares Füllmaterial für zahnärztliche Zwecke, wobei das Material besteht aus:

   - mit Polyphenolen quervernetztes Kollagengel in einer keramischen Trägermatrix, und
   - wässrige oder salzhaltige Pektinlösung bei etwa pH 5 mit einem Essigpuffer.

2. Material nach Anspruch 1, wobei die keramische Trägermatrix aus keramischen Phosphatgranulaten besteht.

3. Material nach Anspruch 2, wobei das keramische Phosphat Hydroxyapatit, Beta-Tri-Calciumphosphat oder Mischungen davon umfasst.

**4.** Material nach einem der Ansprüche 1 bis 3, wobei die keramische Trägermatrix ein Granulat mit einer Größe zwischen 50 und 2000 Mikrometern oder zwischen 100 und 300 Mikrometern ist.

**5.** Material nach einem der Ansprüche 1 bis 4, wobei die Polyphenole ausgewählt sind aus Polyphenolmischungen, die aus Traubenschalen und Traubenkernen extrahiert werden, verwendet im Ist-Zustand oder nach dem Fermentationsprozess und der Weinherstellung und Trocknung.

**6.** Material nach Anspruch 5, wobei die Polyphenolmischungen hydroalkoholische Extrakte aus Schalen und Kernen von Trauben sind.

**7.** Material nach einem der Ansprüche 1 bis 6, wobei das Kollagen Kollagen Typ 1 ist, von Rindern, Schweinen oder Pferden stammt oder rekombinant biotechnologisch hergestellt ist oder aus Pflanzen stammt.

**8.** Material nach einem der Ansprüche 1 bis 7 zur Verwendung in der Medizin.

**9.** Material nach Anspruch 8 zur Verwendung als Füllung von Knochendefekten, wie Defekte aufgrund des Verlusts von Weichgewebe oder Resorption von Knochengewebe um die natürlichen Zähne herum, parodontale Defekte oder Implantant-Schrauben, peri-Implantat-Defekte, um die Größe des Alveolarkamms zu vergrößern, Extraktionsstellen zu füllen und die Kieferhöhle anzuheben.

**10.** Verfahren zur Herstellung des verformbaren Füllmaterials nach einem der Ansprüche 1 bis 7, das die Schritte umfasst:

a) Mischen einer Lösung aus Polyphenolextrakt, das aus Traubentrester erhalten wird, mit einer keramischen Trägermatrix;
b) Trocknen der Mischung aus Schritt a), um ein Granulat zu erhalten;
c) Mischen einer wässrigen Lösung aus Kollagen mit einer wässrigen oder salzhaltigen Pektinlösung bei etwa pH 5 mit einem Essigpuffer;
d) Zugabe der Mischung nach Schritt c) zu dem Granulat nach Schritt b);
e) Mischen der Mischung nach Schritt d), um ein verformbares Füllmaterial zu erhalten.

**11.** Verfahren nach Anspruch 10, wobei die Pektinlösung eine Konzentration zwischen 3 Gewichtsprozent und 10 Gewichtsprozent, bevorzugt zwischen 5 Gewichtsprozent und 8 Gewichtsprozent, aufweist.

**12.** Verfahren nach einem der Ansprüche 10 bis 11, wobei die Kollagenlösung eine Konzentration zwischen 0.05 Gewichtsprozent und 1 Gewichtsprozent, bevorzugt zwischen 0.1 Gewichtsprozent und 0.8 Gewichtsprozent aufweist.

**13.** Verfahren nach einem der Ansprüche 10 bis 12, wobei, in Schritt c), das Gewichtsverhältnis zwischen der Pektinlösung und der Kollagenlösung zwischen 20:80 und 80:20, bevorzugt zwischen 40:60 und 60:40 ist.

**14.** Verfahren nach einem der Ansprüche 10 bis 13, wobei, in Schritt d), das Gewichtsverhältnis zwischen dem Granulat nach Schritt b) und der Mischung nach Schritt c) zwischen 20:80 und 90:10, bevorzugt zwischen 50:50 und 80:20 ist.

**Revendications**

**1.** Matériau de remplissage malléable à usage dentaire, ledit matériau étant constitué de :

- un gel de collagène réticulé avec des polyphénols dans une matrice de support en céramique, et
- une solution aqueuse ou saline de pectine à un pH d'environ 5 avec un tampon acétique.

**2.** Matériau selon la revendication 1, dans lequel la matrice de support en céramique est constituée de granules de phosphate de céramique.

**3.** Matériau selon la revendication 2, dans lequel ledit phosphate de céramique comprend de l'hydroxyapatite, du phosphate de béta-tricalcium ou des mélanges de ceux-ci.

**4.** Matériau selon l'une quelconque des revendications 1 à 3, dans lequel ladite matrice de support en céramique est un granulé d'une taille comprise entre 50 et 2000 micromètres ou entre 100 et 300 micromètres.

5. Matériau selon l'une quelconque des revendications 1 à 4, dans lequel lesdits polyphénols sont choisis parmi des mélanges de polyphénols extraits de peaux de raisin et de pépins de raisin, utilisés en tant que tel ou après le processus de fermentation et la vinification et le séchage.

6. Matériau selon la revendication 5, dans lequel lesdits mélanges polyphénoliques sont des extraits hydroalcooliques des peaux et pépins de raisins.

7. Matériau selon l'une quelconque des revendications 1 à 6, dans lequel ledit collagène est du collagène de type I, d'origine bovine, porcine ou équine, ou du collagène pour la production biotechnologique recombinante ou du collagène provenant de plantes.

8. Matériau selon l'une quelconque des revendications 1 à 7, à usage médical.

9. Matériau selon la revendication 8, à utiliser pour le remplissage de défauts osseux, tels que des défauts dus à la perte de tissus mous ou à la résorption de tissus osseux autour de dents naturelles, de défauts parodontaux ou de vis d'implant, de défauts de péri-implant pour augmenter la taille de la crête alvéolaire, pour remplir des sites d'extraction et pour le relevé du sinus maxillaire.

10. Procédé de préparation du matériau de remplissage malléable selon l'une quelconque des revendications 1 à 7, comprenant les étapes consistant à :

   a) mélanger une solution d'extrait de polyphénol obtenu à partir de marc de raisin avec une matrice de support en céramique ;
   b) sécher le mélange de l'étape a) pour obtenir un granulé ;
   c) mélanger une solution aqueuse de collagène avec une solution aqueuse ou saline de pectine à un pH d'environ 5 avec un tampon acétique ;
   d) ajouter le mélange selon l'étape c) au granulé de l'étape b) ;
   e) mélanger le mélange de l'étape d) pour obtenir un matériau de remplissage malléable.

11. Procédé selon la revendication 10, dans lequel ladite solution de pectine a une concentration en poids comprise entre 3 % et 10 %, de préférence entre 5 % et 8 %.

12. Procédé selon l'une quelconque des revendications 10 à 11, dans lequel ladite solution de collagène a une concentration comprise entre 0,05 % et 1 % en poids, de préférence entre 0,1 % et 0,8 %.

13. Procédé selon l'une quelconque des revendications 10 à 12, dans lequel, dans l'étape c), le rapport de poids entre la solution de pectine et la solution de collagène est compris entre 20:80 et 80:20, de préférence entre 40:60 et 60:40.

14. Procédé selon l'une quelconque des revendications 10 à 13, dans lequel, dans l'étape d), le rapport de poids entre le granulé de l'étape b) et le mélange de l'étape c) est compris entre 20:80 et 90:10, de préférence entre 50:50 et 80:20.

FIG. 1

FIG. 2

# FIG. 3

# FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2295089 A2
- EP 2021044 A2
- US 20020192263 A1
- US 20030206937 A1
- US 7785634 B
- US 5223029 A

**Non-patent literature cited in the description**

- **J GOVINDARAJ ; EMMADI P ; DEEPALAKSHMI ; RAJARAM V ; PRAKASH G ; PUVANAKRISHNAN R.** Protective effect of proanthocyanidins on endotoxin induced experimental periodontitis in rats. *Indian J Exp Biol.,* 04 February 2010, vol. 8 (2), 133-42

- **JOHN SHI ; JIANMEI YU ; JOSEPH POHORLY ; J. CHRISTOPHER YOUNG ; MIKE BRYAN ; YING WU.** Optimization of the extraction of polyphenols from grape seed meal by aqueous ethanol solution. *Food, Agriculture & Environment,* 2003, vol. 1 (2), 42-47